(19) Europäisches Patentamt
European Patent Office
Office européen des brevets

(11) Veröffentlichungsnummer: **0 218 233 B1**

# (12) EUROPÄISCHE PATENTSCHRIFT

(45) Veröffentlichungstag der Patentschrift: **21.03.90**

(51) Int. Cl.⁴: **C07D 335/02**, C07D 309/06, C07D 307/14, C07D 213/53, C07D 493/04, C07D 321/06, A01N 43/08, A01N 43/14, A01N 43/24, A01N 43/40

(21) Anmeldenummer: **86113873.3**

(22) Anmeldetag: **07.10.86**

## (54) Cyclohexenonderivate, Verfahren zu ihrer Herstellung und ihre Verwendung zur Bekämpfung unerwünschten Pflanzenwuchses.

(30) Priorität: **10.10.85 DE 3536117**

(43) Veröffentlichungstag der Anmeldung: **15.04.87 Patentblatt 87/16**

(45) Bekanntmachung des Hinweises auf die Patenterteilung: **21.03.90 Patentblatt 90/12**

(84) Benannte Vertragsstaaten: **AT BE CH DE FR GB IT LI NL SE**

(56) Entgegenhaltungen:
**EP-A- 0 070 370**
**EP-A- 0 071 707**
**EP-A- 0 104 876**
**EP-A- 0 107 156**
**EP-A- 0 136 647**
**EP-A- 0 136 702**
**EP-A- 0 142 741**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Kolassa, Dieter, Dr., Moltkestrasse 8, D-6700 Ludwigshafen(DE)**
Erfinder: **Becker, Rainer, Dr., Im Haseneck 22, D-6702 Bad Duerkheim(DE)**
Erfinder: **Jahn, Dieter, Dr., Burgunder Weg 8, D-6803 Edingen-Neckarhausen(DE)**
Erfinder: **Keil, Michael, Dr., Fontanestrasse 4, D-6713 Freinsheim(DE)**
Erfinder: **Schirmer, Ulrich, Dr., Berghalde 79, D-6900 Heidelberg(DE)**
Erfinder: **Wuerzer, Bruno, Dr., Ruedigerstrasse 13, D-6701 Otterstadt(DE)**
Erfinder: **Meyer, Norbert, Dr., Dossenheimer Weg 22, D-6802 Ladenburg(DE)**

**EP 0 218 233 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Cyclohexenonderivate, Verfahren zu ihrer Herstellung, Herbizide, die diese Verbindungen als Wirkstoff enthalten, sowie ein Verfahren zur Bekämpfung unerwünschten Pflanzenwuchses.

Es ist aus der DE-A 2 439 104 bekannt, Cyclohexenonderivate zur Bekämpfung von unerwünschten Gräsern in breitblättrigen Kulturen anzuwenden. Weiterhin sind in der DE-A 3 121 355, DE-A 3 123 312, DE-A 3 239 071, EP-A 136 702 und EP-A 142 741 heterocyclisch-substituierte Derivate beschrieben.

Es wurden nun neue Cyclohexenonderivate der Formel I

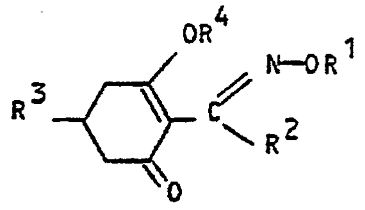

(I),

in der

$R^1$ 2-Fluorethyl, 2-Chlorethyl, But-2-en-1-yl, Methoxymethyl oder Methoxyethyl,

$R^2$ $C_1$–$C_4$-Alkyl, vorzugsweise $C_2$- oder $C_3$-Alkyl,

$R^3$ 2-Isopropyl-1,3-dioxepan-5-yl, Tetrahydrothiopyran-3-yl, Tetrahydropyran-3-yl, Tetrahydropyran-4-yl, 3-Methyltetrahydropyran-4-yl, Pyrid-3-yl, Tetrahydrofuran-3-yl, oder 4a,7,8,8a-Tetrahydro-2H,5H-pyrano[4,3-b]pyran-3-yl, und

$R^4$ Wasserstoff, $C_1$–$C_{20}$-Alkylcarbonyl, $C_2$–$C_{20}$-Alkenylcarbonyl, das vorzugsweise jeweils eine C-C-Doppelbindung aufweist, Benzoyl, das gegebenenfalls durch $C_1$–$C_8$-Alkyl substituiert ist, $C_1$–$C_4$-Trialkylsilyl, $C_1$–$C_4$-Alkylsulfonyl, Phenylsulfonyl, das gegebenenfalls durch $C_1$–$C_4$-Alkyl substituiert ist, $C_1$–$C_4$-Dialkylphosphono oder $C_1$–$C_4$-Dialkylthiophosphono bedeuten, sowie

Salze dieser Verbindung gefunden.

Die neuen Cyclohexenonderivate zeigen eine gute herbizide Wirkung, vorzugsweise gegen Arten aus der Familie oder Gräser (Gramineen).

Die Verbindungen der Formel I können in mehreren tautomeren Formen auftreten, die alle vom Patentanspruch umfaßt werden. Beispielsweise können die Verbindungen mit $R^4$ = Wasserstoff u.a. in folgenden tautomeren Formen auftreten:

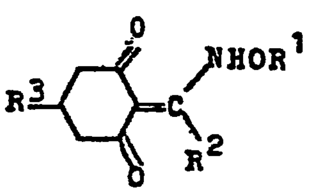

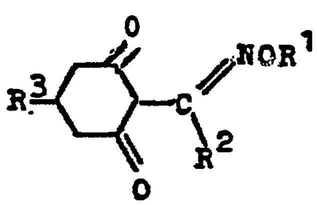

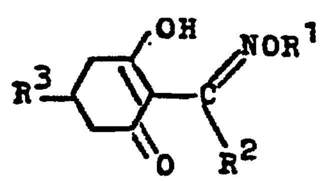

Für den Fall, daß $R^1$ But-2-en-1-yl bedeutet, ist darunter sowohl das E- als auch das Z-Isomere zu verstehen.

$R^2$ in Formel I steht beispielsweise für Methyl, Ethyl, n-Propyl, Isopropyl, n-Butyl, sec-Butyl, Isobutyl oder tert-Butyl.

$R^4$ in Formel I steht beispielsweise für Wasserstoff; Acetyl, Propionyl, Acroyl, Butyryl, Isobutyryl, Pivaloyl, Valeroyl, Caproyl, Caprinoyl, Lauroyl, Palmitoyl, Stearoyl, Oleoyl; Benzoyl, 4-Methylbenzoyl, 4-Hexylbenzoyl; Trimethylsilyl, Triethlsilyl; Methylsulfonyl, Ethylsulfonyl; Benzoylsulfonyl, 4-Methylphenylsulfonyl; Diethylphosphono oder Diethylthiophosphono.

Als Salze der Verbindungen der Formel I kommen landwirtschaftlich brauchbare Salze, beispielsweise die Alkalimetallsalze, insbesondere die Kalium- oder Natriumsalze, Erdalkalimetallsalze, insbesondere Calcium- und Magnesiumsalze, Mangan-, Kupfer-, Zink- oder Eisensalze sowie Ammonium-, Sulfonium- und Phosphoniumsalze in Betracht.

Die neuen Verbindungen der Formel I können durch Umsetzung von Tricarbonylverbindungen der Formel II

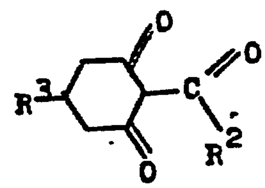 (II),

in der $R^2$ und $R^3$ jeweils die obengenannte Bedeutung besitzen, zunächst mit einer Ammoniumverbindung der Formel $R^1O\text{-}NH_3Y$, in der $R^1$ die obengenannte Bedeutung hat und Y für ein Anion, beispielsweise für ein Halogenid, wie Fluorid, Chlorid, Bromid oder Jodid, für Carbonat oder für Sulfat steht, und anschließend, für den Fall, daß $R^4$ nicht für Wasserstoff steht, durch Umsetzung mit einer Verbindung $R^4X$, in der $R^4$, mit Ausnahme von Wasserstoff, die obengenannte Bedeutung besitzt und X für eine Austrittsgruppe steht, gegebenenfalls in Anwesenheit eines inerten Lösungsmittels erhalten werden.

Die Reaktion mit der Ammoniumverbindung führt man zweckmäßigerweise in heterogener Phase in einem inerten Verdünnungsmittel bei einer Temperatur zwischen O und 8O °C oder zwischen O °C und dem Siedepunkt des Reaktionsgemisches in Gegenwart einer Base durch. Geeignete Basen sind beispielsweise die Carbonate, Hydrogencarbonate, Acetate, Alkanolate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Als inerte Verdünnungsmittel für diesen Reaktionsschritt sind beispielsweise Dimethylsulfoxid, Alkohole, wie Methanol, Ethanol oder Isopropanol, Benzol, gegebenenfalls chlorierte Kohlenwasserstoffe, wie Chloroform, Dichlorethan, Hexan oder Cyclohexan; Carbonsäureester, wie Essigsäureethylester; oder Ether, wie Dioxan oder Tetrahydrofuran geeignet.

Die Umsetzung ist nach wenigen Stunden beendet. Das Reaktionsprodukt kann dann durch Einengen der Mischung, Zugabe von Wasser und Extraktion mit einem unpolaren Lösungsmittel, wie Methylenchlorid und Abdestillieren des Lösungsmittels unter vermindertem Druck isoliert werden.

Anstatt mit einer Ammoniumverbindung können die Tricarbonylverbindungen der Formel II zunächst auch mit einem Hydroxylamin der Formel $R^1O\text{-}NH_2$, in der $R^1$ die obengenannte Bedeutung hat, in inerten Verdünnungsmitteln bei einer Temperatur zwischen O °C und dem Siedepunkt des Reaktionsgemisches, insbesondere zwischen 15 und 7O °C, umgesetzt werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol oder Cyclohexanol; gegebenenfalls chlorierte Kohlenwasserstoffe, wie Hexan, Cyclohexan, Methylenchlorid, Toluol oder Dichlorethan; Carbonsäureester, wie Essigsäureethylester, Nitrile, wie Acetonitril; oder cyclische Ether, wie Tetrahydrofuran.

Für den Fall, daß $R^4$ in Formel I für Wasserstoff steht, gelant man nach der Umsetzung mit der Ammoniumverbindung bzw. mit dem Hydroxylamin bereits zum Zielprodukt.

Für den Fall jedoch, daß $R^4$ in Formel I eine andere Bedeutung als Wasserstoff besitzt, schließt sich noch eine weitere Umsetzung mit einer Verbindung der Formel $R^4$ X an, in der $R^4$, mit der Ausnahme von Wasserstoff, die obengenannte Bedeutung besitzt und X für eine Austrittsgruppe (z.B. Chlor, Brom oder Hydroxyl) steht.

Bei diesem Reaktionsschritt handelt es sich um eine an sich bekannte Veresterungs- bzw. Silylierungsreaktion. Solche Umsetzungen werden im allgemeinen bei einer Temperatur von O bis 15O°C, gegebenenfalls in Anwesenheit eines inerten Lösungsmittels (z.B. Toluol oder Chlorbenzol) und gegebenenfalls in Anwesenheit einer Hilfsbase (z.B. Kaliumcarbonat) durchgeführt.

Die oben genannten Alkalimetallsalze der Cyclohexenonderivate der Formel I können durch Behandeln dieser Verbindungen mit Natrium- oder Kaliumhydroxid in wäßriger Lösung oder in einem organischen Lösungsmittel, wie Methanol, Ethanol oder Aceton, erhalten werden. Auch Natrium- und Kaliumalkanolate können als Basen dienen.

Die anderen Metallsalze, z. B. die Mangan-, Kupfer-, Zink-, Eisen-, Calcium- und Magnesiumsalze können aus den Natriumsalzen durch Umsetzung mit den entsprechenden Metallchloriden in wäßriger Lösung hergestellt werden. Die Ammonium-, Sulfonium- und Phosphoniumsalze lassen sich aus den neuen Verbindungen der Formel I mittels Ammonium- oder Phosphoniumhydroxiden gegebenenfalls in wäßriger Lösung herstellen.

Die Tricarbonylverbindungen der Formel II können aus Cyclohexenonen der Formel III, die auch in der tautomeren Form IIIa vorliegen können,

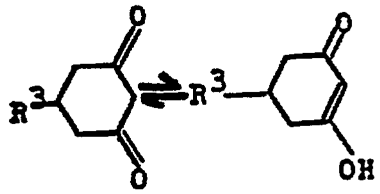

(III) (IIIa)

nach literaturbekannten Methoden durch Umsetzung mit Carbonsäureanhydriden (Tetrahedron Letters, 29, 2491 (1975) hergestellt werden.

Es ist auch möglich, die Tricarbonylverbindungen der Formel II über die Zwischenstufe der Enolester herzustellen. Diese fallen bei der Umsetzung der Cyclohexenone der Formel III (eventuell als Isomerengemische) an und können in Gegenwart von Imidazol- oder Pyridinderivaten umgelagert werden (JP-A-36052/1979).

Zu den Verbindungen der Formel III gelangt man ebenfalls nach literaturbekannten Verfahren, wie dies aus folgendem Schema hervorgeht:

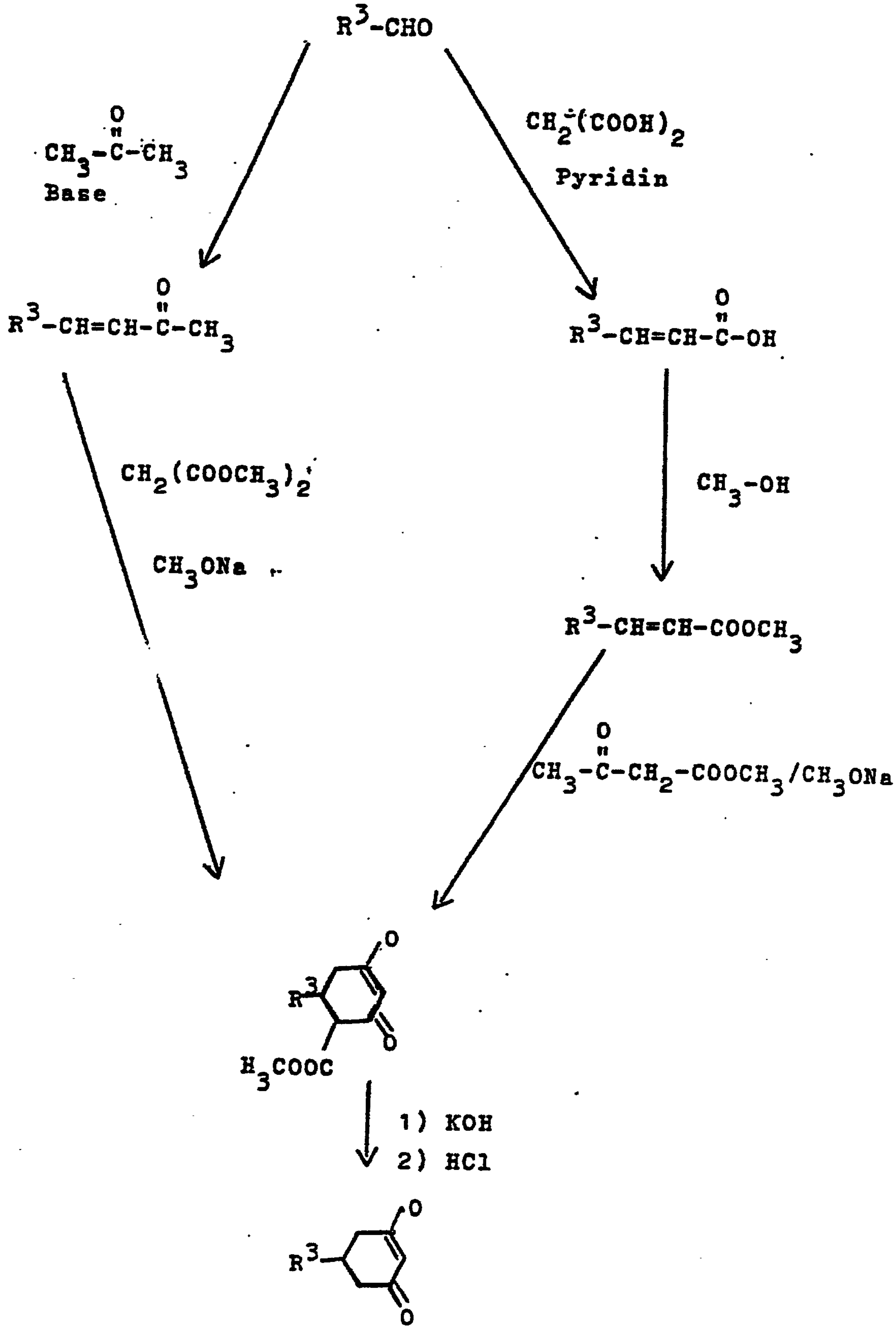
Base
Pyridin
$CH_3ONa$
$CH_3$-OH
1) KOH
2) HCl

Die im obigen Schema aufgeführten Aldehyde der allgemeinen Formel $R^3$-CHO sind nach literaturbekannten Verfahren zugänglich, beispielsweise durch Oxidation der entsprechenden Alkohole, Reduktion von Carbonsäurederivaten oder Hydroformylierung von Olefinen.

Die folgenden Beispiele sollen die Herstellung der neuen Cyclohexenonderivate näher erläutern. In den Beispielen verhalten sich Gewichtsteile zu Volumenteilen wie Kilogramm zu Liter.

Die $^1$H-NMR-Spektren wurden in Deuterochloroform als Lösungsmittel mit Tetramethylsilan als innerem Standard aufgenommen. Die $^1$H-chemischen Verschiebungen sind jeweils in $\delta$ [ppm] angegeben. Für die Signalstruktur wurden folgende Abkürzungen benützt:
s = Singulett, d = Dublett, t = Triplett, q = Quartett, m = Multiplett, stärkestes Signal.

In den Beispielen ist der But-2-en-1-yl-rest in verkürzter Weise als "2-Butenyl" aufgeführt. Die Isomerenform ist jeweils angegeben.

Beispiel 1

7,3 Gewichtsteile 2-Acetyl-5-(3-tetrahydrothiopyranyl)-cyclohexan-1,3-dion, 3,6 Gewichtsteile 2-Butenyloxyammoniumchlorid und 2,4 Gewichtsteile Natriumhydrogencarbonat wurden in 1OO Volumenteilen Methanol 16 Stunden bei Raumtemperatur gerührt.
Das Lösungsmittel wurde unter vermindertem Druck abdestilliert, der Rückstand mit einem Gemisch aus jeweils 5O Volumenteilen Wasser und Dichlormethan gerührt und die wäßrige Phase einmal mit 5O Volumenteilen Dichlormethan extrahiert. Die vereinigten organischen Phasen wurden mit Wasser gewaschen, über Natriumsulfat getrocknet und das Lösungsmittel unter vermindertem Druck abdestilliert. Man erhielt 2-1-[2-Butenyloxyimino)ethyl]-3-hydroxy-5-(3-tetrahydrothiopyranyl)-cyclohex-2-en-1-on als Feststoff mit Schmelzpunkt 65 °C (Verbindung Nr. 2).

Beispiel 2

86 Gewichtsteile 2-Butyryl-5-(3-tetrahydrothiopyranyl)-cyclohexan-1,3-dion und 2.6 Gewichtsteile 2-Butenyloxyamin wurden 16 Stunden bei Raumtemperatur in Dichlormethan gerührt. Die Lösung wird mit 5 gew.%iger Salzsäure und Wasser gewaschen, über Natriumsulfat getrocknet und das Lösungsmittel unter vermindertem Druck abdestilliert. Man erhielt 2-[1-(2-Butenyloxyimino)butyl]-3-hydroxy-5-(3-tetrahydrothiopyranyl)-cyclohex-2-en-1-on als Öl (Verbindung Nr. 14).

Beispiel 3

5 Gewichtsteile 2-Butyryl-5-(tetrahydrothiopyran-3-yl)cyclohexan-1,3-dion, 2,31 Gewichtsteile 2-Fluorethyloxyammoniumchlorid und 1,68 Gewichtsteile Natriumhydrogencarbonat wurden in 100 Volumenteilen Methanol bei Raumtemperatur 16 Stunden gerührt. Das Lösungsmittel wurde unter vermindertem Druck abdestilliert, der Rückstand in Dichlormethan aufgenommen und im Verhältnis 1:1 mit Wasser versetzt. Die wäßrige Phase wurde abgetrennt und mit Dichlormethan extrahiert. Die vereinigten organischen Phasen wurden über Natriumsulfat getrocknet und unter vermindertem Druck konzentriert. Man erhielt 5,3 Teile (85%) 2-[1-(2-Fluorethyloxyimino)butyl]-3-hydroxy-5-(tetrahydrothiopyran-3-yl)-cyclohex-2-en-1-on als leicht gelb gefärbtes Öl (Verbindung Nr. 13).

Weitere Cyclohexenonderivate sind in den Tabellen 1 und 2 aufgeführt. Ihre Herstellung geschieht in analoger Weise.

In Tabelle 1 steht der Rest $R^4$ für Wasserstoff.

Tabelle 1

| Verbindung-Nr. | $R^3$ | $R^2$ | $R^1$ | $^1$H-NMR-Daten/Schmp. [°C] |
|---|---|---|---|---|
| 1 | Tetrahydrothiopyran-3-yl | Methyl | 2-Fluorethyl | |
| 2 | Tetrahydrothiopyran-3-yl | Methyl | 2-Butenyl (E) | 2.3(s), 4.45(d), 5.65(m) |
| 3 | Tetrahydrothiopyran-3-yl | Methyl | 2-Butenyl (Z) | |
| 4 | Tetrahydrothiopyran-3-yl | Methyl | 2-Chlorethyl | |
| 5 | Tetrahydrothiopyran-3-yl | Methyl | Methoxymethyl | |
| 6 | Tetrahydrothiopyran-3-yl | Methyl | 2-Methoxyethyl | |
| 7 | Tetrahydrothiopyran-3-yl | Ethyl | 2-Fluorethyl | |
| 8 | Tetrahydrothiopyran-3-yl | Ethyl | 2-Butenyl (E) | 1.1(t), 1.75(d), 4.5(d) |
| 9 | Tetrahydrothiopyran-3-yl | Ethyl | 2-Butenyl (Z) | |
| 10 | Tetrahydrothiopyran-3-yl | Ethyl | 2-Chlorethyl | |
| 11 | Tetrahydrothiopyran-3-yl | Ethyl | Methoxymethyl | |
| 12 | Tetrahydrothiopyran-3-yl | Ethyl | 2-Methoxyethyl | |
| 13 | Tetrahydrothiopyran-3-yl | n-Propyl | 2-Fluorethyl | 1.0(t), 2.9(m), 4.6(t), 4.75(t) |
| 14 | Tetrahydrothiopyran-3-yl | n-Propyl | 2-Butenyl (E) | 1.75 (d), 2.35(s), 4.40(d) |
| 15 | Tetrahydrothiopyran-3-yl | n-Propyl | 2-Butenyl (Z) | |
| 16 | Tetrahydrothiopyran-3-yl | n-Propyl | 2-Chlorethyl | 0.95(6), 3.75(t), 4.33(t) |
| 17 | Tetrahydrothiopyran-3-yl | n-Propyl | Methoxymethyl | 0.97(t), 3.44(s),5.04(s) |
| 18 | Tetrahydrothiopyran-3-yl | n-Propyl | 2-Methoxyethyl | 0.97(t), 3.40(s), 4.23(m) |
| 19 | Tetrahydropyran-4-yl | Methyl | 2-Fluorethyl | |
| 20 | Tetrahydropyran-4-yl | Methyl | 2-Butenyl (E) | |

Tabelle 1 (Forts.)

| Verbindung -Nr. | $R^3$ | $R^2$ | $R^1$ | $^1$H-NMR-Daten/Schmp. [°C] |
|---|---|---|---|---|
| 21 | Tetrahydropyran-4-yl | Methyl | 2-Butenyl (Z) | |
| 22 | Tetrahydropyran-4-yl | Methyl | 2-Chlorethyl | |
| 23 | Tetrahydropyran-4-yl | Methyl | Methoxymethyl | |
| 24 | Tetrahydropyran-4-yl | Methyl | 2-Methoxyethyl | |
| 25 | Tetrahydropyran-4-yl | Ethyl | 2-Fluorethyl | |
| 26 | Tetrahydropyran-4-yl | Ethyl | 2-Butenyl (E) | 1.1(t), 1.8(d), 3,35(t) |
| 27 | Tetrahydropyran-4-yl | Ethyl | 2-Butenyl (Z) | |
| 28 | Tetrahydropyran-4-yl | Ethyl | 2-Chlorethyl | |
| 29 | Tetrahydropyran-4-yl | Ethyl | Methoxymethyl | 1.17(t), 3.44(s), 4.02(m), 5.05(m) |
| 30 | Tetrahydropyran-4-yl | Ethyl | 2-Methoxyethyl | 1.12(t), 3.41(s), 4.23(m) |
| 31 | Tetrahydropyran-4-yl | n-Propyl | 2-Fluorethyl | |
| 32 | Tetrahydropyran-4-yl | n-Propyl | 2-Butenyl (E) | 0.95(t), 2.9(d), 4.5(d) |
| 33 | Tetrahydropyran-4-yl | n-Propyl | 2-Butenyl (Z) | |
| 34 | Tetrahydropyran-4-yl | n-Propyl | 2-Chlorethyl | |
| 35 | Tetrahydropyran-4-yl | n-Propyl | Methoxymethyl | 0.98(t), 3.44(s), 4.02(m), 5.05(m) |
| 36 | Tetrahydropyran-4-yl | n-Propyl | 2-Methoxyethyl | 0.97(t), 2.87(t), 3.41(s) |
| 37 | Tetrahydropyran-3-yl | Methyl | 2-Fluorethyl | |
| 38 | Tetrahydropyran-3-yl | Methyl | 2-Butenyl (E) | |
| 39 | Tetrahydropyran-3-yl | Methyl | 2-Butenyl (Z) | |
| 40 | Tetrahydropyran-3-yl | Methyl | 2-Chlorethyl | |

Tabelle 1 (Forts.)

| Verbindung-Nr. | $R^3$ | $R^2$ | $R^1$ | $^1H$-NMR-Daten/Schmp. [°C] |
|---|---|---|---|---|
| 41 | Tetrahydropyran-3-yl | Methyl | Methoxymethyl | |
| 42 | Tetrahydropyran-3-yl | Methyl | 2-Methoxyethyl | |
| 43 | Tetrahydropyran-3-yl | Ethyl | 2-Fluorethyl | |
| 44 | Tetrahydropyran-3-yl | Ethyl | 2-Butenyl (E) | |
| 45 | Tetrahydropyran-3-yl | Ethyl | 2-Butenyl (Z) | |
| 46 | Tetrahydropyran-3-yl | Ethyl | 2-Chlorethyl | |
| 47 | Tetrahydropyran-3-yl | Ethyl | Methoxymethyl | |
| 48 | Tetrahydropyran-3-yl | Ethyl | 2-Methoxyethyl | |
| 49 | Tetrahydropyran-3-yl | n-Propyl | 2-Fluorethyl | 0.95(t), 3.95(m), 4.6(t) 4.75(t) |
| 50 | Tetrahydropyran-3-yl | n-Propyl | 2-Butenyl (E) | 0.95(t), 3.2(t), 4.45(d) |
| 51 | Tetrahydropyran-3-yl | n-Propyl | 2-Butenyl (Z) | |
| 52 | Tetrahydropyran-3-yl | n-Propyl | 2-Chlorethyl | 0.92(t), 3.7(t), 4.3(t) |
| 53 | Tetrahydropyran-3-yl | n-Propyl | Methoxymethyl | |
| 54 | Tetrahydropyran-3-yl | n-Propyl | 2-Methoxyethyl | |
| 55 | 3-Methyl-tetrahydropyran-4-yl | Methyl | 2-Fluorethyl | |
| 56 | 3-Methyl-tetrahydropyran-4-yl | Methyl | 2-Butenyl (E) | |
| 57 | 3-Methyl-tetrahydropyran-4-yl | Methyl | 2-Butenyl (Z) | |
| 58 | 3-Methyl-tetrahydropyran-4-yl | Methyl | 2-Chlorethyl | |
| 59 | 3-Methyl-tetrahydropyran-4-yl | Methyl | Methoxymethyl | |
| 60 | 3-Methyl-tetrahydropyran-4-yl | Methyl | 2-Methoxyethyl | |

Tabelle 1 (Forts.)

| Verbindung-Nr. | $R^3$ | $R^2$ | $R^1$ | $^1H$-NMR-Daten/Schmp. [°C] |
|---|---|---|---|---|
| 61 | 3-Methyl-tetrahydropyran-4-yl | Ethyl | 2-Fluorethyl | |
| 62 | 3-Methyl-tetrahydropyran-4-yl | Ethyl | 2-Butenyl (E) | 0.80(d), 1.75(d), 4.5(d) |
| 63 | 3-Methyl-tetrahydropyran-4-yl | Ethyl | 2-Butenyl (Z) | |
| 64 | 3-Methyl-tetrahydropyran-4-yl | Ethyl | 2-Chlorethyl | |
| 65 | 3-Methyl-tetrahydropyran-4-yl | Ethyl | Methoxymethyl | |
| 66 | 3-Methyl-tetrahydropyran-4-yl | Ethyl | 2-Methoxyethyl | |
| 67 | 3-Methyl-tetrahydropyran-4-yl | n-Propyl | 2-Fluorethyl | |
| 68 | 3-Methyl-tetrahydropyran-4-yl | n-Propyl | 2-Butenyl (E) | |
| 69 | 3-Methyl-tetrahydropyran-4-yl | n-Propyl | 2-Butenyl (Z) | |
| 70 | 3-Methyl-tetrahydropyran-4-yl | n-Propyl | 2-Chlorethyl | |
| 71 | 3-Methyl-tetrahydropyran-4-yl | n-Propyl | Methoxymethyl | |
| 72 | 3-Methyl-tetrahydropyran-4-yl | n-Propyl | 2-Methoxyethyl | |
| 73 | Tetrahydrofuran-3-yl | Methyl | 2-Fluorethyl | |
| 74 | Tetrahydrofuran-3-yl | Methyl | 2-Butenyl (E) | |
| 75 | Tetrahydrofuran-3-yl | Methyl | 2-Butenyl (Z) | |
| 76 | Tetrahydrofuran-3-yl | Methyl | 2-Chlorethyl | |
| 77 | Tetrahydrofuran-3-yl | Methyl | Methoxymethyl | |
| 78 | Tetrahydrofuran-3-yl | Methyl | 2-Methoxyethyl | |
| 79 | Tetrahydrofuran-3-yl | Ethyl | 2-Fluorethyl | |
| 80 | Tetrahydrofuran-3-yl | Ethyl | 2-Butenyl (E) | 1.8(d), 3.45(t), 4.45(d) |

Tabelle 1 (Forts.)

| Verbindung-Nr. | $R^3$ | $R^2$ | $R^1$ | $^1H$-NMR-Daten/Schmp. [°C] |
|---|---|---|---|---|
| 81 | Tetrahydrofuran-3-yl | Ethyl | 2-Butenyl (Z) | |
| 82 | Tetrahydrofuran-3-yl | Ethyl | 2-Chlorethyl | |
| 83 | Tetrahydrofuran-3-yl | Ethyl | Methoxymethyl | |
| 84 | Tetrahydrofuran-3-yl | Ethyl | 2-Methoxyethyl | |
| 85 | Tetrahydrofuran-3-yl | n-Propyl | 2-Fluorethyl | |
| 86 | Tetrahydrofuran-3-yl | n-Propyl | 2-Butenyl (E) | 0.95(t), 1.5(m), 5.65(m) |
| 87 | Tetrahydrofuran-3-yl | n-Propyl | 2-Butenyl (Z) | |
| 88 | Tetrahydrofuran-3-yl | n-Propyl | 2-Chlorethyl | |
| 89 | Tetrahydrofuran-3-yl | n-Propyl | Methoxymethyl | |
| 90 | Tetrahydrofuran-3-yl | n-Propyl | 2-Methoxyethyl | |
| 91 | Pyrid-3-yl | Methyl | 2-Fluorethyl | |
| 92 | Pyrid-3-yl | Methyl | 2-Butenyl (E) | |
| 93 | Pyrid-3-yl | Methyl | 2-Butenyl (Z) | |
| 94 | Pyrid-3-yl | Methyl | 2-Chlorethyl | |
| 95 | Pyrid-3-yl | Methyl | Methoxymethyl | |
| 96 | Pyrid-3-yl | Methyl | 2-Methoxyethyl | |
| 97 | Pyrid-3-yl | Ethyl | 2-Fluorethyl | |
| 98 | Pyrid-3-yl | Ethyl | 2-Butenyl (E) | |
| 99 | Pyrid-3-yl | Ethyl | 2-Butenyl (Z) | |
| 100 | Pyrid-3-yl | Ethyl | 2-Chlorethyl | |

Tabelle 1 (Forts.)

| Verbindung-Nr. | $R^3$ | $R^2$ | $R^1$ | $^1$H-NMR-Daten/Schmp. [°C] |
|---|---|---|---|---|
| 101 | Pyrid-3-yl | Ethyl | Methoxymethyl | |
| 102 | Pyrid-3-yl | Ethyl | 2-Methoxyethyl | |
| 103 | Pyrid-3-yl | n-Propyl | 2-Fluorethyl | 85 - 87 |
| 104 | Pyrid-3-yl | n-Propyl | 2-Butenyl (E) | 0.95(t), 1.7(d), 8.5(m) |
| 105 | Pyrid-3-yl | n-Propyl | 2-Butenyl (Z) | |
| 106 | Pyrid-3-yl | n-Propyl | 2-Chlorethyl | |
| 107 | Pyrid-3-yl | n-Propyl | Methoxymethyl | |
| 108 | Pyrid-3-yl | n-Propyl | 2-Methoxyethyl | |
| 109 | 4a,7,8,8a-Tetrahydro-2H-5H-pyrano[4,3-b]pyran-3-yl | Methyl | 2-Fluorethyl | |
| 110 | 4a,7,8,8a-Tetrahydro-2H-5H-pyrano[4,3-b]pyran-3-yl | Methyl | 2-Butenyl (E) | |
| 111 | 4a,7,8,8a-Tetrahydro-2H-5H-pyrano[4,3-b]pyran-3-yl | Methyl | 2-Butenyl (Z) | |
| 112 | 4a,7,8,8a-Tetrahydro-2H-5H-pyrano[4,3-b]pyran-3-yl | Methyl | 2-Chlorethyl | |
| 113 | 4a,7,8,8a-Tetrahydro-2H-5H-pyrano[4,3]b]pyran-3-yl | Methyl | Methoxymethyl | |
| 114 | 4a,7,8,8a-Tetrahydro-2H-5H-pyrano[4,3-b]pyran-3-yl | Methyl | 2-Methoxyethyl | |
| 115 | 4a,7,8,8a-Tetrahydro-2H-5H-pyrano[4,3-b]pyran-3-yl | Ethyl | 2-Fluorethyl | |
| 116 | 4a,7,8,8a-Tetrahydro-2H-5H-pyrano[4,3-b]pyran-3-yl | Ethyl | 2-Butenyl (E) | |
| 117 | 4a,7,8,8a-Tetrahydro-2H-5H-pyrano[4,3-b]pyran-3-yl | Ethyl | 2-Butenyl (Z) | |
| 118 | 4a,7,8,8a-Tetrahydro-2H-5H-pyrano[4,3-b]pyran-3-yl | Ethyl | 2-Chlorethyl | |
| 119 | 4a,7,8,8a-Tetrahydro-2H-5H-pyrano[4,3-b]pyran-3-yl | Ethyl | Methoxymethyl | |
| 120 | 4a,7,8,8a-Tetrahydro-2H-5H-pyrano[4,3-b]pyran-3-yl | Ethyl | 2-Methoxyethyl | |

Tabelle 1 (Forts.)

| Verbindung-Nr. | $R^3$ | $R^2$ | $R^1$ | $^1$H-NMR-Daten/Schmp. [°C] |
|---|---|---|---|---|
| 121 | 4a,7,8,8a-Tetrahydro-2H-5H-pyrano[4,3-b]pyran-3-yl | n-Propyl | 2-Fluorethyl | 0.95(t), 2.9(m), 4.6(t), 4.75(t) |
| 122 | 4a-7,8,8a-Tetrahydro-2H-5H-pyrano[4,3-b]pyran-3-yl | n-Propyl | 2-Butenyl (E) | 1.75(d), 2.9(m), 5.6(m) |
| 123 | 4a,7,8,8a-Tetrahydro-2H-5H-pyrano[4,3-b]pyran-3-yl | n-Propyl | 2-Butenyl (Z) | |
| 124 | 4a,7,8,8a-Tetrahydro-2H-5H-pyrano[4,3-b]pyran-3-yl | n-Propyl | 2-Chlorethyl | |
| 125 | 4a,7,8,8a-Tetrahydro-2H-5H-pyrano[4,3-b]pyran-3-yl | n-Propyl | Methoxymethyl | |
| 126 | 4a,7,8,8a-Tetrahydro-2H-5H-pyrano[4,3-b]pyran-3-yl | n-Propyl | 2-Methoxyethyl | 0.97(t), 3.38(s), 5.35(s) |
| 127 | 2-Isopropyl-1,3-dioxepan-5-yl | Methyl | 2-Fluorethyl | |
| 128 | 2-Isopropyl-1,3-dioxepan-5-yl | Methyl | 2-Butenyl (E) | |
| 129 | 2-Isopropyl-1,3-dioxepan-5-yl | Methyl | 2-Butenyl (Z) | |
| 130 | 2-Isopropyl-1,3-dioxepan-5-yl | Methyl | 2-Chlorethyl | |
| 131 | 2-Isopropyl-1,3-dioxepan-5-yl | Methyl | Methoxymethyl | |
| 132 | 2-Isopropyl-1,3-dioxepan-5-yl | Methyl | 2-Methoxyethyl | |
| 133 | 2-Isopropyl-1,3-dioxepan-5-yl | Ethyl | 2-Fluorethyl | |
| 134 | 2-Isopropyl-1,3-dioxepan-5-yl | Ethyl | 2-Butenyl (E) | 0.9(m), 1.115(t), 4.45(d) |
| 135 | 2-Isopropyl-1,3-dioxepan-5-yl | Ethyl | 2-Butenyl (Z) | |
| 136 | 2-Isopropyl-1,3-dioxepan-5-yl | Ethyl | 2-Chlorethyl | |
| 137 | 2-Isopropyl-1,3-dioxepan-5-yl | Ethyl | Methoxymethyl | |
| 138 | 2-Isopropyl-1,3-dioxepan-5-yl | n-Propyl | 2-Methoxyethyl | 1.16(t), 3.42(s), 4.22(m) |
| 139 | 2-Isopropyl-1,3-dioxepan-5-yl | n-Propyl | 2-Fluorethyl | |
| 140 | 2-Isopropyl-1,3-dioxepan-5-yl | n-Propyl | 2-Butenyl (E) | |

<u>Tabelle 2</u>

| Verbindung-Nr. | $R^1$ | $R^2$ | $R^3$ | $R^4$ | $^1$H-NMR-Daten |
|---|---|---|---|---|---|
| 141 | 2-Fluorethyl | n-Propyl | 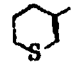 | Butyryl | 0.95(t,3H), 2.85(d,2H), 4.22(t,1H), 4.33(t,1H), 4.58(t,1H), 4.74(t,1H) |
| 142 | 2-Fluorethyl | n-Propyl | 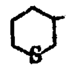 | Acetyl | 0.9(t,3H), 1.4(m,2H), 2.17(s,3H), 4.23(t,1H), 4.34(t,1H), 4.53(t,1H), 4.7(t,1H) |
| 143 | 2-Fluorethyl | n-Propyl | 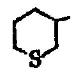 | Benzoyl | 0.85(t,3H), 1.4(m,2H), 4.08(t,1H), 4.17(t,1H), 4.32(t,1H), 4.5(t,1H), 7.5(m2H), 7.62(m,1H), 8.03(d,2H) |
| 144 | 2-Fluorethyl | n-Propyl | 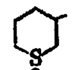 | Caproyl | 0.9(m,6H), 4.2(t,1H), 4.33(t,1H), 4.5(t,1H), 4.65(t,1H) |
| 145 | 2-Fluorethyl | n-Propyl | 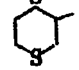 | Pivaloyl | 0.9(t,3H), 1.2(s,9H), 4.22(t,1H), 4.33(t,1H), 4.52(t,1H), 4.67(t, 1H) |
| 146 | 2-Fluroethyl | n-Propyl | 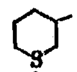 | Lauroyl | 0.9(m,6H), 1.3(m,12H), 4.22(t,1H), 4.35(t,1H), 4,53(t,1H), 4.68(t, 1H) |
| 147 | 2-Fluorethyl | n-Propyl | 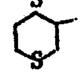 | 4-Hexyl-benzoyl | 0.85(t,6H), 1.3(m,12H), 4.1(t,1H), 4.2(t,1H), 4.35(t,1H, 4.5(t,1H), 7.25(d,2H), 7.92(d,2H) |
| 148 | 2-Fluorethyl | n-Propyl | 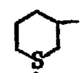 | Caprinoyl | 0.85(m,6H), 1.3(m,18H), 4.2(t,1H), 4.32(t,1H), 4.52(t,1H), 4.68(t, 1H) |
| 149 | 2-Methoxyethyl | n-Propyl | 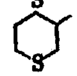 | Butyryl | 0.86(t,3H), 0.95(t,3H), 1.36(m,2H), 3.39(s,3H), 3.6(t,2H), 4.2(t,2H) |
| 150 | 2-Methoxyethyl | n-Propyl | 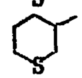 | Benzoyl | 0.85(t,3H), 1.4(m,2H), 3.17(s,3H), 3.2(t,2H), 4,05(t,2H), 7.45(m,2H), 7.62(m,1H), 8.05(d,2H) |
| 151 | 2-Methoxyethyl | n-Propyl | 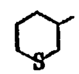 | Pivaloyl | 0.88(t,3H), 1.23(s,9H), 3.38(s, 3H), 3.6(t,2H), 4.2(t,2H) |

Die Cyclohexenonderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen O,1 und 95 Gew.%, vorzugsweise zwischen O,5 und 9O Gew.% Wirkstoff.

Beispiele für Formulierungen sind:

I. Man vermischt 9O Gewichtsteile der Verbindung Nr. 134 mit 1O Gewichtsteilen N-Methyl- -pyrrolidon und erhält eine Lösung, die zur Anwendung in Form kleinster Tropfen geeignet ist.

II. 2O Gewichtsteile der Verbindung Nr. 5O werden in einer Mischung gelöst, die aus 8O Gewichtsteilen Xylol, 1O Gewichtsteilen des Anlagerungsproduktes von 8 bis 1O Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 4O Mol Ethylenoxid an 1 Mol Ricinusöl besteht. Durch Ausgießen und feines Verteilen der Lösung in 1OO OOO Gewichtsteilen Wasser erhält man eine wäßrige Dispersion, die O,O2 Gew.% des Wirkstoffs enthält.

III. 2O Gewichtsteile der Verbindung Nr. 2 werden in einer Mischung gelöst, die aus 4O Gewichtsteilen Cyclohexanon, 3O Gewichtsteilen Isobutanol, 2O Gewichtsteilen des Anlagerungsproduktes von 7 Mol Ethylenoxid an 1 Mol Isooctylphenol und 1O Gewichtsteilen des Anlagerungsproduktes von 4O Mol Ethylenoxid an 1 Mol Ricinusöl besteht. Durch Eingießen und feines Verteilen der Lösung in 1OO OOO Gewichtsteilen Wasser erhält man eine wäßrige Dispersion, die O,O2 Gew.% des Wirkstoffs enthält.

IV. 2O Gewichtsteile des Wirkstoffs Nr. 1O4 werden in einer Mischung gelöst, die aus 25 Gewichtsteilen Cyclohexanon, 65 Gewichtsteilen einer Mineralölfraktion vom Siedepunkt 21O bis 28O°C und 1O Gewichtsteilen des Anlagerungsproduktes von 4O Mol Ethylenoxid an 1 Mol Ricinusöl besteht. Durch Eingießen und feines Verteilen der Lösung in 1OO OOO Gewichtsteilen Wasser erhält man eine wäßrige Dispersion, die O,O2 Gew.% des Wirkstoffs enthält.

V. 2O Gewichtsteile des Wirkstoffs Nr. 14 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 6O Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 122 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 50 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. 40 Gewichtsteile des Wirkstoffs Nr. 2 werden in 60 Gewichtsteilen einer Mischung, die aus 93 Gew.% Xylol und 7 Gew.% des Anlagerungsproduktes von 8 Mol Ethylenoxid an 1 Mol Nonylphenol besteht, gelöst. Man erhält eine Lösung, die 40 Gew.% des Wirkstoffs enthält.

Die neuen Herbizide besitzen eine gute herbizide Wirkung vorzugsweise gegen Arten aus der Familie der Gräser (Gramineen). Sie sind verträglich und somit selektiv in breitblättrigen Kulturen sowie in monokotylen Gewächsen, welche nicht zu den Gramineen zählen. Es sind auch Wirkstoffe darunter, die zusätzlich herbizide Wirkung gegen breitblättrige Pflanzen zeigen und auch solche, die zur nicht selektiven Bekämpfung oder Unterdrückung unerwünschter Vegetation geeignet sind.

Die Applikation der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Jahreszeit, Zielpflanzen und Wachstumsstadium 0,015 bis 3 kg/ha, vorzugsweise 0,05 bis 0,5 kg/ha.

Die Wirkung der Cyclohexenonderivate der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 Gew.-% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,015 bis 3,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen.

| Lateinischer Name: | Deutscher Name: |
|---|---|
| Alopecurus myosuroides | Ackerfuchsschwanz |
| Avena fatua | Flughafer |
| Beta vulgaris | Zuckerrübe |
| Centaurea cyanus | Kornblume |
| Digitaria sanguinalis | Blutfingerhirse |
| Echinochloa crus galli | Hühnerhirse |
| Glycine max | Sojabohnen |
| Hordeum vulgare | Gerste |
| Ipomoea spp. | Prunkwindearten |
| Mercurialis annua | Einjähriges Bingelkraut |
| Oryza sativa | Reis |
| Setaria italica | Kolbenhirse |
| Sinapis alba | Weißer Senf |
| Sorghum bicolor | Mohrenhirse |
| Triticum aestivum | Weizen |
| Zea mays | Mais |

Die herbizide Wirkung der beispielhaft ausgewählten Wirkstoffe Nr. 134, 122, 5O und 1O4 gegen verbreitete Ungräser und Ausfallkulturen ist derjenigen chemisch eng verwandter Derivate deutlich überlegen. Als Vergleichsmittel dienten Herbizide, die Wirkstoffe enthielten, die aus der DE-A-3 34O 265, DE-A-3 239 O71, DE-A-3 123 312 und DE-A-3 121 355 bekannt sind.

Beispielsweise eignen sich die Wirkstoffe Nr. 2 und 14 in einer Aufwandmenge von 3 kg/ha zur Bekämpfung unerwünschter grasartiger Vegetation in breitblättrigen Kulturen, z.B. Sojabohnen und Zuckerrüben, ohne diese nennenswert zu beeinflussen. Sie besitzen eine höhere herbizide Aktivität als bekannten Vergleichswirkstoffe, die aus der DE-A-3 121 355 bekannt sind.

Der beispielhaft ausgewählte Wirkstoff Nr. 2 zeigt die Möglichkeit des Einsatzes der neuen Verbindungen zur Unterdrückung breitblättriger unerwünschter Vegetation. Im Vor- und Nachauflaufverfahren wird eine deutlich bessere herbizide Wirkung erzielt als mit den bekannten Wirkstoffen.

Im Vergleich mit bekannten Verbindungen aus GB 2 137 2OO und EP-125 O94 entsprechenden Verbindungen zeigt beispielsweise der Wirkstoff Nr. 49 bei niedriger Aufwandmenge gegen unerwünschte Gräse und Ausfallweizen eine deutlich stärkere herbizide Wirkung bei sehr guter Verträglichkeit für Soja.

Schon bei geringen Aufwandmengen eignen sich die Wirkstoffe Nr. 8, 26 und 62 zur Bekämpfung eines breiten Spektrums an unerwünschten grasartigen Pflanzen, ohne die Kulturpflanze Soja zu schädigen.

In Anbetracht des erfassbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden.
In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elais guineensis | Ölpalme |
| Fragaria vesca | Erdbeere |
| Glycine max | Soyabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Mentha piperita | Pfefferminze |
| Musa spp. | Obst- und Mehlbanane |
| Nicothiana tabacum (N. rustica) | Tabak |
| Olea europaea | Ölbaum |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I mit zahlreichen Vertretern anderer herbizider oder wachstumsregulierender Wirkstoffgruppen gemischt und gemeinsam ausgebracht werden. Beispielsweise kommen als

Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenonderivate der Formel I, allein oder in Kombination mit anderen Herbiziden, auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Cyclohexenonderivate der Formel I

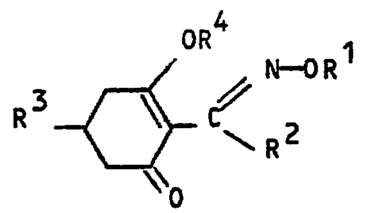

(I),

in der
$R^1$ 2-Fluorethyl, 2-Chlorethyl, But-2-en-1-yl, Methoxymethyl oder Methoxyethyl,
$R^2$ $C_1$-$C_4$-Alkyl,
$R^3$ 2-Isopropyl-1,3-dioxepan-5-yl, Tetrahydrothiopyran-3-yl, Tetrahydropyran-3-yl, Tetrahydropyran-4-yl, 3-Methyltetrahydropyran-4-yl, Pyrid-3-yl, Tetrahydrofuran-3-yl oder 4a, 7, 8, 8a-Tetrahydro-2H, 5H-pyrano[4,3-b]pyran-3-yl
und
$R^4$ Wasserstoff, $C_1$-$C_{20}$-Alkylcarbonyl, $C_2$-$C_{20}$-Alkenylcarbonyl, Benzoyl, das gegebenenfalls durch $C_1$-$C_8$-Alkyl substituiert ist, $C_1$-$C_4$-Trialkylsilyl, $C_1$-$C_4$-Alkylsulfonyl, Phenylsulfonyl, das gegebenenfalls durch $C_1$-$C_4$-Alkyl substituiert ist, $C_1$-$C_4$-Dialkylphosphono oder $C_1$-$C_4$-Dialkylthiophosphono bedeuten,
und
Salze dieser Verbindungen.

2. Verfahren zur Herstellung eines Cyclohexenonderivates der Formel I

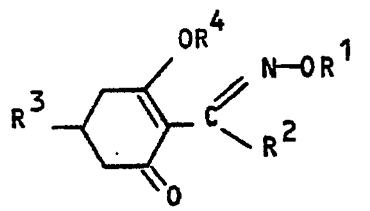

(I),

in der
$R^1$ 2-Fluorethyl, 2-Chlorethyl, But-2-en-1-yl, Methoxymethyl oder Methoxyethyl,
$R^2$ $C_1$-$C_4$-Alkyl,
$R^3$ 2-Isopropyl-1,3-dioxepan-5-yl, Tetrahydrothiopyran-3-yl, Tetrahydropyran-3-yl, Tetrahydropyran-4-yl, 3-Methyltetrahydropyran-4-yl, Pyrid-3-yl, Tetrahydrofuran-3-yl oder 4a, 7, 8, 8a-Tetrahydro-2H, 5H-pyrano[4,3-b]pyran-3-yl
und
$R^4$ Wasserstoff, $C_1$-$C_{20}$-Alkylcarbonyl, $C_2$-$C_{20}$-Alkenylcarbonyl, Benzoyl, das gegebenenfalls durch $C_1$-$C_8$-Alkyl substituiert ist, $C_1$-$C_4$-Trialkylsilyl, $C_1$-$C_4$-Alkylsulfonyl, Phenylsulfonyl, das gegebenenfalls durch $C_1$-$C_4$-Alkyl substituiert ist, $C_1$-$C_4$-Dialkylphosphono oder $C_1$-$C_4$-Dialkylthiophosphono bedeuten,
und
Salze dieser Verbindungen, dadurch gekennzeichnet, daß man eine Tricarbonylverbindung der Formel II

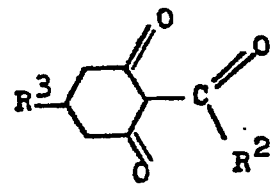 (II),

in der
$R^2$ und $R^3$ die oben genannten Bedeutungen besitzen, zunächst

a) mit einer Ammoniumverbindung der Formel $R^1O-NH_3Y$, in der $R^1$ die oben genannten Bedeutungen hat und Y ein Anion bedeutet, in einem inerten Verdünnungsmittel gegebenenfalls in Gegenwart von Wasser bei einer Temperatur zwischen O und 8O °C in Gegenwart einer Base oder

b) mit einem gegebenenfalls in wäßriger Lösung vorliegenden Hydroxylamin der Formel $R^1O-NH_2$, in der $R^1$ die oben genannten Bedeutungen besitzt, in einem inerten Lösungsmittel und anschließend, für den Fall, daß $R^4$ nicht für Wasserstoff steht, mit einer Verbindung $R^4X$, in der $R^4$, mit Ausnahme von Wasserstoff, die oben genannte Bedeutung besitzt und X für eine Austrittsgruppe steht, gegebenenfalls in Anwesenheit eines inerten Lösungsmittel umsetzt.

3. Herbizid, enthaltend ein Cyclohexenonderivat der Formel I gemäß Anspruch 1.

4. Herbizid, enthaltend einen inerten Zusatzstoff und ein Cyclohexenonderivat der Formel I

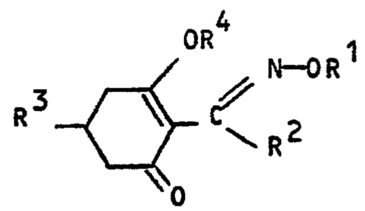 (I),

in der
$R^1$ 2-Fluorethyl, 2-Chlorethyl, But-2-en-1-yl, Methoxymethyl oder Methoxyethyl,
$R^2$ $C_1$-$C_4$-Alkyl,
$R^3$ 2-Isopropyl-1,3-dioxepan-5-yl, Tetrahydrothiopyran-3-yl, Tetrahydropyran-3-yl, Tetrahydropyran-4-yl, 3-Methyltetrahydropyran-4-yl, Pyrid-3-yl, Tetrahydrofuran-3-yl oder 4a, 7, 8, 8a-Tetrahydro-2H, 5H-pyrano[4,3-b]pyran-3-yl
und
$R^4$ Wasserstoff, $C_1$-$C_{20}$-Alkylcarbonyl, $C_2$-$C_{20}$-Alkenylcarbonyl, Benzoyl, das gegebenenfalls durch $C_1$-$C_8$-Alkyl substituiert ist, $C_1$-$C_4$-Trialkylsilyl, $C_1$-$C_4$-Alkylsulfonyl, Phenylsulfonyl, das gegebenenfalls durch $C_1$-$C_4$-Alkyl substituiert ist, $C_1$-$C_4$-Dialkylphosphono oder $C_1$-$C_4$-Dialkylthiophosphono bedeuten,
und
Salze dieser Verbindungen.

5. Verfahren zur Bekämpfung unerwünschten Pflanzenwachstums, dadurch gekennzeichnet, daß man die unerwünschten Pflanzen oder die von unerwünschten Pflanzenwachstum freizuhaltende Fläche mit einer herbizid wirksamen Menge eines Cyclohexenonderivates der Formel I

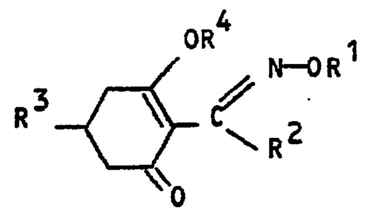 (I),

in der
$R^1$ 2-Fluorethyl, 2-Chlorethyl, But-2-en-1-yl, Methoxymethyl oder Methoxyethyl,
$R^2$ $C_1$-$C_4$-Alkyl,
$R^3$ 2-Isopropyl-1,3-dioxepan-5-yl, Tetrahydrothiopyran-3-yl, Tetrahydropyran-3-yl, Tetrahydropyran-4-yl, 3-Methyltetrahydropyran-4-yl, Pyrid-3-yl, Tetrahydrofuran-3-yl oder 4a, 7, 8, 8a-Tetrahydro-2H, 5H-pyrano[4,3-b]pyran-3-yl
und
$R^4$ Wasserstoff, $C_1$-$C_{20}$-Alkylcarbonyl, $C_2$-$C_{20}$-Alkenylcarbonyl, Benzoyl, das gegebenenfalls durch $C_1$-$C_8$-Alkyl substituiert ist, $C_1$-$C_4$-Trialkylsilyl, $C_1$-$C_4$-Alkylsulfonyl, Phenylsulfonyl, das gegebenenfalls durch $C_1$-$C_4$-Alkyl substituiert ist, $C_1$-$C_4$-Dialkylphosphono oder $C_1$-$C_4$-Dialkylthiophosphono bedeuten,
und

Salze dieser Verbindungen behandelt.

6. Cyclohexenonderivat gemäß Anspruch 1, dadurch gekennzeichnet, daß $R^1$ But-2-en-1-yl, $R^2$ Methyl, Ethyl oder Propyl und $R^4$ Wasserstoff bedeutet und $R^3$ die in Anspruch 1 genannten Bedeutungen hat.

7. 2-[1-(2-Butenyloxi-imino)ethyl]-3-hydroxy-5-(3-tetrahydrothiopyranyl)-Cyclohex-2-en-1-on.

**Claims**

1. A cyclohexenone derivative of the formula I

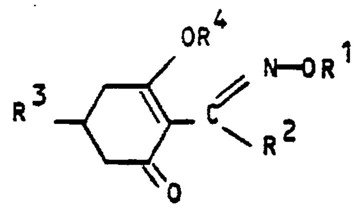

(I),

where $R^1$ is 2-fluoroethyl, 2-chloroethyl, but-2-en-yl, methoxymethyl or methoxyethyl, $R_2$ is $C_1$–$C_4$-alkyl, $R^3$ is 2-isopropyl-1,3-dioxepan-5-yl, tetrahydrothiopyran-3-yl, tetrahydropyran-3-yl, tetrahydropyran-4-yl, 3-methyl-tetrahydropyran-4-yl, pyrid-3-yl, tetrahydrofuran-3-yl or 4a,7,8,8a-tetrahydro-2H, 5H-pyrano[4,3-b]pyran-3-yl, and $R^4$ is hydrogen, $C_1$–$C_{20}$-alkylcarbonyl, $C_2$–$C_{20}$-alkenylcarbonyl, benzoyl which is unsubstituted or substituted by $C_1$–$C_8$-alkyl, $C_1$–$C_4$-trialkylsilyl, $C_1$–$C_4$-alkylsulfonyl, phenylsulfonyl which is unsubstituted or substituted by $C_1$–$C_4$-alkyl, $C_1$–$C_4$-dialkylphosphono or $C_1$–$C_4$-dialkylthiophosphono or salts of these compounds.

2. A process for the preparation of a cyclohexenone derivative of the formula I

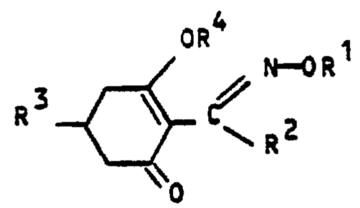

(I),

where $R^1$ is 2-fluoroethyl, 2-chloroethyl, but-2-en-yl, methoxymethyl or methoxyethyl, $R^2$ is $C_1$–$C_4$-alkyl, $R^3$ is 2-isopropyl-1,3-dioxepan-5-yl, tetrahydrothiopyran-3-yl, tetrahydropyran-3-yl, tetrahydropyran-4-yl, 3-methyl-tetrahydropyran-4-yl, pyrid-3-yl, tetrahydrofuran-3-yl or 4a,7,8,8a-tetrahydro-2H, 5H-pyrano[4,3-b]pyran-3-yl, and $R^4$ is hydrogen, $C_1$–$C_{20}$-alkylcarbonyl, $C_2$–$C_{20}$-alkenylcarbonyl, benzoyl which is unsubstituted or substituted by $C_1$–$C_8$-alkyl, $C_1$–$C_4$-trialkylsilyl, $C_1$–$C_4$-alkylsulfonyl, phenylsulfonyl which is unsubstituted or substituted by $C_1$–$C_4$-alkyl, $C_1$–$C_4$-dialkylphosphono or $C_1$–$C_4$-dialkylthiophosphono or salts of these compounds, wherein a tricarbonyl compound of the formula II

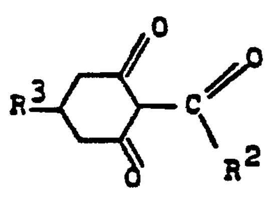

(II),

where $R^2$ and $R^3$ have the above meanings, is first reacted

a) with an ammonium compound of the formula $R^1O\text{-}NH_3Y$ in which $R^1$ has the above meanings and Y is an anion, in an inert diluent in the presence or absence of water at from 0 to 80°C in the presence of a base, or

b) with a hydroxylamine, if desired present in aqueous solution, of the formula $R^1O\text{-}NH_2$ in which $R^1$ has the above meanings, in an inert solvent and then, if $R^4$ is not hydrogen, with a compound $R^4X$ in which $R^4$ has the above meanings, with the exception of hydrogen, and X is a leaving group, in the presence or absence of an inert solvent.

3. A herbicide containing a cyclohexenone derivative of the formula I as claimed in claim 1.

4. A herbicide containing an inert additive and a cyclohexenone derivative of the formula I

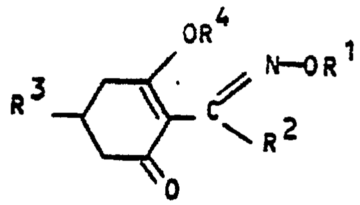

(I),

where $R^1$ is 2-fluoroethyl, 2-chloroethyl, but-2-en-yl, methoxymethyl or methoxyethyl, $R^2$ is $C_1$–$C_4$-alkyl, $R^3$ is 2-isopropyl-1,3-dioxepan-5-yl, tetrahydrothiopyran-3-yl, tetrahydropyran-3-yl, tetrahydropyran-4-yl, 3-methyl-tetrahydropyran-4-yl, pyrid-3-yl, tetrahydrofuran-3-yl or 4a,7,8,8a-tetrahydro-2H, 5H-pyrano[4,3-b]pyran-3-yl, and $R^4$ is hydrogen, $C_1$–$C_{20}$-alkylcarbonyl, $C_2$–$C_{20}$-alkenylcarbonyl, benzoyl which is unsubstituted or substituted by $C_1$–$C_4$-alkyl, $C_1$–$C_4$-trialkylsilyl, $C_1$–$C_4$-alkylsulfonyl, phenylsulfonyl which is unsubstituted or substituted by $C_1$–$C_8$-alkyl, $C_1$–$C_4$-dialkylphosphono or $C_1$–$C_4$-dialkylthiophosphono or salts of these compounds.

5. A process for controlling undesirable plant growth, wherein the undesirable plants or the area to be kept free of undesirable plant growth are or is treated with a herbicidally active amount of a cyclohexenone derivative of the formula I

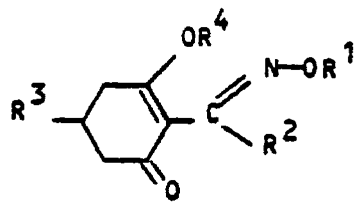

(I),

where $R^1$ is 2-fluoroethyl, 2-chloroethyl, but-2-en-yl, methoxymethyl or methoxyethyl, $R^2$ is $C_1$–$C_4$-alkyl, $R^3$ is 2-isopropyl-1,3-dioxepan-5-yl, tetrahydrothiopyran-3-yl, tetrahydropyran-3-yl, tetrahydropyran-4-yl, 3-methyl-tetrahydropyran-4-yl, pyrid-3-yl, tetrahydrofuran-3-yl or 4a,7,8,8a-tetrahydro-2H, 5H-pyrano[4,3-b]pyran-3-yl, and $R^4$ is hydrogen, $C_1$–$C_{20}$-alkylcarbonyl, $C_2$–$C_{20}$-alkenylcarbonyl, benzoyl which is unsubstituted or substituted by $C_1$–$C_8$-alkyl, $C_1$–$C_4$-trialkylsilyl, $C_1$–$C_4$-alkylsulfonyl, phenylsulfonyl which is unsubstituted or substituted by $C_1$–$C_4$-alkyl, $C_1$–$C_4$-dialkylphosphono or $C_1$–$C_4$-dialkylthiophosphono or salts of these compounds.

6. A cyclohexenone derivative as claimed in claim 1, wherein $R^1$ is but-2-en-yl, $R^2$ is methyl, ethyl or propyl, $R^4$ is hydrogen and $R^3$ has the meanings specified in claim 1.

7. 2-[1-(2-butenyloxyimino)-ethyl]-3-hydroxy-5-(3-tetrahydrothiopyranyl)-cyclohex-2-en-1-one.

## Revendications

1. Dérivés de cyclohexenone de formule I

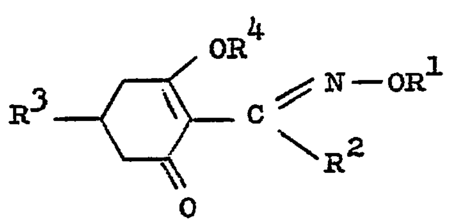

(I),

dans laquelle

$R^1$ représente 2-fluoréthyle, 2-chloréthyle, but-2-en-yle, méthoxyméthyle ou méthoxyéthyle,

$R^2$, alkyle en $C_1$–$C_4$,

$R^3$, 2-isopropyle-1,3-dioxepan-5-yle, tétrahydrothiopyran-3-yle, tétrahydropyran-3-yle, tétrahydropyran-4-yle, 3-méthyltétrahydropyran-4-yle, pyrid-3-yle, tétrahydrofuran-3-yle ou 4a,7,8,8a-tétrahydro-2H,5H-pyrano[4,3-b]pyran-3-yle et

$R^4$, hydrogène, alkylcarbonyle en $C_1$–$C_{20}$, alcénylcarbonyle en $C_2$–$C_{20}$, benzoyle, qui est éventuellement substitué par alkyle en $C_1$–$C_8$, trialkylsilyle en $C_1$–$C_4$, alkylsulfonyle en $C_1$–$C_4$, phénylsulfonyle, qui est éventuellement substitué par alkyle en $C_1$–$C_4$ dialkylphosphono en $C_1$–$C_4$ ou dialkylthiophosphono en $C_1$–$C_4$ et

sels de ces composés.

2. Procédé de préparation d'un dérivé de cyclohexenone de formule I

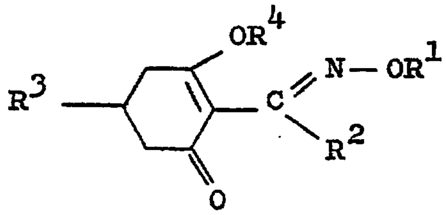 (I),

dans laquelle

$R^1$ représente 2-fluoréthyle, 2-chloréthyle, but-2-en-yle, méthoxyméthyle ou méthoxyle ou méthoxyéthyle,

$R^2$, alkyle en $C_1$–$C_4$,

$R^3$, 2-isopropyle-1,3-dioxepan-5-yle, tétrahydrothiopyran-3-yle, tétrahydropyran-3-yle, tétrahydropyran-4-yle, 3-méthyltétrahydropyran-4-yle, pyrid-3-yle, tétrahydrofuran-3-yle ou 4a,7,8,8a-tétrahydro-2H,5H-pyrano[4,3-b]pyran-3-yle et

$R^4$, hydrogène, alkylcarbonyle en $C_1$–$C_{20}$, alcénylcarbonyle en $C_2$–$C_{20}$, benzoyle, qui est éventuellement substitué par alkyle en $C_1$–$C_8$, trialkylsilyle en $C_1$–$C_4$, alkylsulfonyle en $C_1$–$C_4$, phénylsulfonyle, qui est éventuellement substitué par alkyle en $C_1$–$C_4$ dialkylphosphono en $C_1$–$C_4$ ou dialkylthiophosphono en $C_1$–$C_4$ et

sels de ces composés,

caractérisé par le fait qu'on fait réagir un composé tricarbonyle de formule II

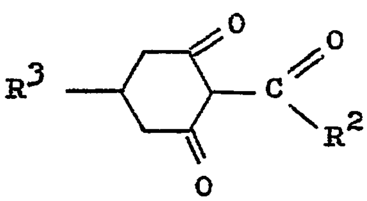 (II),

dans laquelle

$R^2$ et $R^3$ ont les significations sus-indiquées d'abord

a) avec un composé d'ammonium de formule $R^1O\text{-}NH_3Y$, dans laquelle $R^1$ a la signification sus-indiquée et y représente un anion, dans un diluant inerte, éventuellement en présence d'eau, à une température comprise entre 0 et 80°C, en présence d'une base, ou

b) avec une hydroxylamine, se trouvant éventuellement en solution aqueuse, de formule $R^1O\text{-}NH_2$, dans laquelle $R^1$ a la signification sus-indiquée, dans un solvant inerte, et ensuite, dans le cas où $R^4$ n'est pas mis pour hydrogène, avec un composé $R^4X$, dans lequel $R^4$, à l'exception d'hydrogène, a la signification sus-indiquée, et X est mis pour un groupe éliminable, éventuellement en présence d'un solvant inerte.

3. Herbicide contenant un dérivé de cyclohexenone de formule I selon la revendication 1.

4. Herbicide contenant un additif inerte et un dérivé de cyclohexenone de formule I

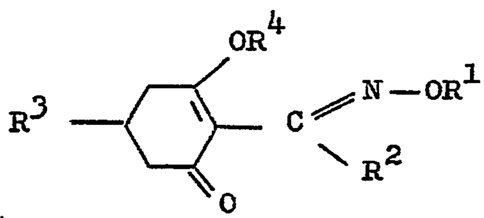 (I),

dans laquelle

$R^1$ représente 2-fluoréthyle, 2-chloréthyle, but-2-en-yle, méthoxyméthyle ou méthoxyéthyle,

$R^2$, alkyle en $C_1$–$C_4$,

$R^3$, 2-isopropyle-1,3-dioxepan-5-yle, tétrahydrothiopyran-3-yle, tétrahydropyran-3-yle, tétrahydropyran-4-yle, 3-méthyltétrahydropyran-4-yle, pyrid-3-yle, tétrahydrofuran-3-yle ou 4a,7,8,8a-tétrahydro-2H,5H-pyrano[4,3-b]pyran-3-yle et

$R^4$, hydrogène, alkylcarbonyle en $C_1$–$C_{20}$, alcénylcarbonyle en $C_2$–$C_{20}$, benzoyle, qui est éventuellement substitué par alkyle en $C_1$–$C_8$, trialkylsilyle en $C_1$–$C_4$, alkylsulfonyle en $C_1$–$C_4$, phénylsulfonyle, qui est éventuellement substitué par alkyle en $C_1$–$C_4$ dialkylphosphono en $C_1$–$C_4$ ou dialkylthiophosphono en $C_1$–$C_4$ et

sels de ces composés.

5. Procédé de lutte contre la croissante de plates indésirables, caractérisé par le fait que l'on traite les plantes indésirables ou les surfaces à maintenir exemptes d'une croissace de plantes indésirables avec une quantité efficace du point de vue herbicide d'un dérivé de cyclohexenone de formule I

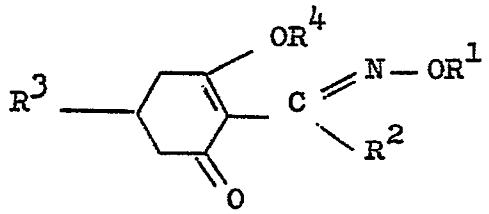 (I),

dans laquelle

$R^1$ représente 2-fluoréthyle, 2-chloréthyle, but-2-en-yle, méthoxyméthyle ou méthoxyéthyle,

$R^2$, alkyle en $C_1$–$C_4$,

$R^3$, 2-isopropyle-1,3-dioxepan-5-yle, tétrahydrothiopyran-3-yle, tétrahydropyran-3-yle, tétrahydropyran-4-yle, 3-méthyltétrahydropyran-4-yle, pyrid-3-yle, tétrahydrofuran-3-yle ou 4a,7,8,8a-tétrahydro-2H,5H-pyrano[4,3-b]pyran-3-yle et

$R^4$, hydrogène, alkylcarbonyle en $C_1$–$C_{20}$, alcénylcarbonyle en $C_2$–$C_{20}$, benzoyle, qui est éventuellement substitué par alkyle en $C_1$–$C_8$, trialkylsilyle en $C_1$–$C_4$, alkylsulfonyle en $C_1$–$C_4$, phénylsulfonyle, qui est éventuellement substitué par alkyle en $C_1$–$C_4$ dialkylphosphono en $C_1$–$C_4$ ou dialkylthiophosphono en $C_1$–$C_4$ et

sels de ces composés.

6. Dérivé de cyclohexenone selon la revendication 1, caractérisé par le fait que $R^1$ signifie but-2-en-2-yle, $R^2$ méthyle, éthyle ou propyle et $R^4$ hydrogène et $R^3$ a les significations indiquées en revendication 1.

7. 2-[1-(2-butenyloxy-imino)éthyl]-3-hydroxy-5-(3-tétrahydrothiopyranyl)cyclohex-2-en-1-one.